# EUROPEAN PATENT APPLICATION

(11) **EP 3 961 352 A1**
(43) Date of publication of application: **02.03.2022**
(21) Application number: 20192659.9
(22) Date of filing: 25.08.2020
(51) Int. Cl.: G06F 3/01, G16H 40/20

(54) **SYSTEM AND METHOD FOR MONITORING A SURFACE**

(71) Applicant: Hitachi, Ltd., Tokyo 100-8280 (JP)
(72) Inventor: AhKun, Leonard Martin, London, EC2Y 5AJ (GB); YOSHIDA, Eiji, London, EC2Y 5AJ (GB)
(74) Representative: MERH-IP Matias Erny Reichl Hoffmann Patentanwälte PartG mbB

(57) **Abstract**

The present disclosure relates to a monitoring system (100) for monitoring a surface. The monitoring system (100) comprises an input interface (101) configured to receive a reference image from a camera (109) which captures a specific location and a stream of pictures captured at the specific location, a processor (103), an output interface (107), and a memory unit (105) coupled to the processor (103) and storing program instructions that are executed by the processor (103). The program instructions comprise commands that configure the processor (103) to identify a person and its pose in the stream of pictures received by the input interface (101), to extract a limb position of the person relative to the reference image received by the input interface (101), based on the pose, to generate an indicator that indicates a touched position based on the extracted limb position, and to output the indicator via the output interface (107).

## Description

The present disclosure relates to a monitoring system, a monitoring method, and a cleaning system.

### Background

Infectious diseases caused by viruses can be spread by micro droplets suspended in the air and contaminate surfaces. Thus, it is preferable for people to avoid touching contaminated surfaces.

Since a virus contaminating a surface is invisible for the human eye, adequate cleaning of contaminated surfaces is difficult.

EP 3 291 191 A1 describes a suspicious person report system which tracks a suspicious person using two cameras in a particular area, such as an airport.

EP 2 571 264 A1 describes a surveillance system for estimating locations of passengers and their private belongings or goods in public transport by video.

### Problem and Solution

It is an object of the herein described disclosure to provide a solution to the problem of automatically detecting and/or cleaning surfaces contaminated by a virus. This problem is solved by the subject-matter of the independent claims.

In particular, the present disclosure proposes to identify a person and its pose in a stream of pictures and to generate an indicator that indicates, in particular visualizes, a touched position, i.e. position where a touching between a person and an object or location occurred, based on the extracted pose and the reference image. The indicator may be used by a sanitizing technician or by a robot to clean a location indicated by the indicator.

The following aspects are in particular provided:
According to a first aspect, there is provided a monitoring system for monitoring a surface. The monitoring system may comprise an input interface configured to receive a reference image from a camera (or from a memory) which captures a specific location/predetermined area and a stream of pictures captured at the specific location/predetermined area, a processor, an output interface, and/or a memory unit coupled to the processor and storing program instructions that are executed by the processor. The program instructions may comprise commands that configure the processor to identify a person and its pose in the stream of pictures received by the input interface, to extract a limb position of the person relative to the reference image received by the input interface, based on the pose, to generate an indicator that indicates a touched position based on the extracted limb position, and/or to output the indicator via the output interface. As a non limiting example said indicator may be general position information regarding a touched position. The indicator may also be for example a set of pixels that is blurred and/or shown in a specific color. Additionally, or alternatively, the indicator may comprise information for identifying a touched position,

In the context of the present disclosure, a pose such as a gesture of a person in which one or more limbs of the person, in particular a hand or wrist of the person, are held in a specific way. A pose may also be a movement of one or more limbs of a person resulting in a touching of an object, for example.

In the context of the present disclosure, an input interface may be a communication interface, such as cable port or a wireless interface for communication over a network or for direct communication with a device, such as a camera, for example.

In the context of the present disclosure, an output interface may be a communication interface, such as a cable port or a wireless interface for communication over a network or for direct communication with a device, such as a display, for example. An output interface may comprise an output device or may be integrated into an output device, such as a display, for example.

In the context of the present disclosure, a limb may be any movable body part of a person's body, in particular a hand or a wrist.

The monitoring system described herein provides for an indicator that indicates a touched position. The indicator may be a set of pixels that is blurred and/or shown in a specific color. Additionally, or alternatively, the indicator may comprise information for identifying a touched position, such as a coordinate or a description of the touched position, for example.

The monitoring system may be used to monitor a sanitizing state of objects in a railway, at an airport, at a hospital, in public areas, such as a tourist spot, by monitoring dirty surfaces.

The monitoring system disclosed herein may be a server that is connected to a plurality of cameras providing the monitoring system with at least one reference picture and one or more streams of pictures for monitoring persons.

The monitoring system disclosed herein may be a server that is connected to a plurality of output devices, such as mobile phones or other display devices in order to output indicators for indicating objects and/or locations to be sanitized.

The monitoring system disclosed herein may be a closed system comprising a camera providing the monitoring system with at least one reference picture and streams of pictures for monitoring persons and an output device for outputting a number, in particular a plurality of indicators for indicating objects and/or locations to be sanitized.

The number of indicators may be displayed on a display device, such as a monitor or video glasses, as a coordinate of the reference image and/or an object in a specific location shown in the reference image. Alternatively, or additionally, the indicator may comprise control commands for controlling a robot to sanitize a location and/or an object in a specific location shown in the reference image.

Technically advantageously, the system allows to provide an automated detection of possibly contaminated areas/locations and the data evaluation is reduced in complexity such that a reliable detection result can be output quickly to a plurality of users and/or other computers. The operational effort of the computation process is reduced by the reduced complexity of the detection process itself which relies on the automatic evaluation of the recorded images. It was found by the inventors that infected locations can be detected automatically and reliably by the proposed subject matter described.

According to an example, the reference image includes depth information of an object and/or a location shown in the reference image, and the program instructions comprise commands that configure the processor to generate the indicator in case a difference between a depth position of a limb of the person and depth information of the object and/or location in the reference image is less than a given distance threshold.

A distance threshold setting a maximum distance between a person and an object causing generation of the indicator, enables the identification of touched positions that are likely to be contaminated. Thus, an information overload of a target, such as a sanitizing technician or a robot, by too many indicators, can be avoided. According to an example, the indicator threshold is determined based on information of a contamination of a particular virus, such as COVID-19, for example.

According to another example, the program instructions may comprise commands that configure the processor to calculate a score based on the difference between a depth position of a limb of the person and depth information of the at least one object and/or location in the reference image, and to choose an indicator from a plurality of indicators according to the calculated score.

By using a score, such as a number representing a difference between a depth position of a limb of the person and depth information of the at least one object and/or location in the reference image, a likelihood of contamination caused by touching of an object by a person can be quantified. This quantification may be used to select an indicator representing the likelihood of contamination caused by the touching, such as a colored indicator. For instance, a green indicator may indicate a small score, representing a small likelihood of contamination, and a red indicator may indicate a high score, representing a high likelihood of contamination.

According to another example, the program instructions may comprise commands that configure the processor to calculate a depth map of the reference image by comparing coordinates of objects to coordinates of empty space in the background of the reference image, or to calculate the depth map by using information provided by a depth sensor.

A depth map may be a three-dimensional coordinate system comprising depth information of all or a selected number of objects and/or locations shown in a reference image. The depth map may be superimposed by coordinates of limbs of a person extracted from a stream of pictures received by the processing system disclosed herein, in order to identify a touching between the person and the objects and/or locations in the reference image.

Depth information, such as coordinates of objects in a three-dimensional space represented in a reference image, can be calculated by the processor of the monitoring system disclosed herein, by using image processing techniques, such as distance calculation to a reference point, for example. Alternatively, sensor information provided by a depth sensor, such as a LIDAR sensor, a RADAR sensor or a stereo-camera may be used to calculate the depth information.

According to another example, the program instructions may comprise commands that configure the processor to generate the indicator in case the difference between a depth position of a limb of the person and depth information of the at least one object and/or location in the reference image is less than a given distance threshold for a time-window that is larger than a given time threshold.

By using a time-window as a criterion to generate the indicator, short touches, that are unlikely to cause a contamination may be excluded from generating an indicator. Thus, the number of indicators is minimized and an information overload of a target, such as a person or a robot, such as a sanitizing technician, by too many indicators, can be avoided.

According to another example, the indicator may comprise at least one indicator information of the following list: a touch marker superimposing the reference image at the touched position, a position information of the touched position, and a quantitative parameter of a sanitized state of the touched position, wherein the quantitative parameter may be a scale parameter determined by a given look-up table that associates a particular quantitative parameter to a particular contact time and/or a particular contact area of the person's limb and the touched position, and a timestamp of a moment a touching of an object by a person was recognized.

A touch marker, such as an arrow or a set of pixels that are blurred and/or colored, enable a user to recognize touched positions fast and easy. Thus, the person can recognize the sanitizing state of a specific location at a glance. In order to indicate whether a specific location is safe, a quantitative parameter, such as a color scale comprising green, yellow and red, wherein green represents a safe state and red represents an unsafe state, or a number scale, such as numbers from "1" to "6", wherein "1" represents a safe state and "6" represents an unsafe state, may be used.

The quantitative parameter may be determined using a look-up table comprising all states of the quantitative parameter and corresponding touch information, such as a number of touches, a number of people that carried out touches, a duration of the touches or a time that has passed since a touch has been carried out by a person.

According to an example, the quantitative parameter is used as an indicator for urgency of a sanitizing procedure. Thus, a surface that has been labelled with an indicator having a quantitative parameter indicating a high number of touches or any other state that makes a contamination very likely, may be ranked at the top of a priority list of objects to be sanitized.

According to another example, the program instructions may comprise commands that configure the processor to adapt the indicator over time according to a mathematical function representing a time period, during which a contamination, with, for example, a virus, becomes harmless, i.e. is not a health risk for a human being anymore.

As contaminations, such as viruses or bacteria, decompose over time, the likelihood of contamination caused by touching of a contaminated object decrease over time as well. In order to minimize the number of sanitizing procedures for objects that are unlikely to contaminate other people, the indicator may be adapted by using a color scale that changes from red over yellow to green over a given time period, for example.

According to another example, the program instructions may comprise commands that configure the processor to identify a particular person by storing a person ID in the memory unit.

By storing an ID of a person in a memory unit, the person can be tracked as the person moves through a building, for example. Thus, a behavior of the person may be analyzed in order to generate characteristics of the person, such as having contact to another person or being isolated.

According to another example, the program instructions may comprise commands that configure the processor to adapt a logic for generating the indicator according to characteristics of the particular person using the person ID.

By storing an ID of a person in a memory unit, information about that person, such as a body temperature, provided by a temperature sensor located at an entrance of a building, for example, may be used to adapt threshold values for generating respective indicators, for that person. Accordingly, an indicator may be generated faster, i.e. a shorter contact period or a larger distance between an object and the person may already generate an indicator.

According to a further aspect, a monitoring method for monitoring a surface may be disclosed. The method may make use of the monitoring system disclosed herein. The monitoring method may comprise a first receiving step for receiving a reference image from a camera which captures a specific location using the input interface, a second receiving step for receiving a stream of pictures captured at the specific location, using the input interface, an identification step for identifying a person and its pose in the stream of pictures received by the input interface, using the processor, an extraction step for extracting a limb position of the person relative to the reference image received by the input interface, based on the pose, using the processor, a generation step for generating an indicator that indicates a touched position based on the extracted limb position, using the processor, and/or an output step for outputting the indicator via the output interface, using the processor.

According to a further aspect, a computer program product may be disclosed. The computer program product having computer program logic arranged to put into effect the monitoring method for monitoring a surface according to the monitoring method disclosed herein, when being executed by a computer system.

According to a further aspect, a cleaning system comprising an embodiment of the monitoring system disclosed herein, and a robot with a sanitizing element. The robot is configured to sanitize an object indicated by an indicator, using the sanitizing element.

The sanitizing element may be a robotic arm equipped with a cleaning device, such as brush, for example. Alternatively, or additionally, the sanitizing element may be a sprayer for spraying a sanitizer.

In particular, the robot is configured to sanitize objects and/or locations indicated by an indicator provided by the monitoring system disclosed herein.

### Brief Description of Drawings

- **Fig. 1**: shows a monitoring system according to an embodiment,
- **Fig. 2**: shows a configuration of the monitoring system according to another embodiment,
- **Fig. 3**: shows a flow chart for detecting a touching of an object by a person, according to an embodiment,
- **Fig. 4**: shows a process for identifying a pose according to an embodiment,
- **Fig. 5**: shows an outputted reference image with indicators calculated according to an embodiment, and
- **Fig. 6**: shows a cleaning system according to an embodiment.

### Detailed Description of Exemplary Embodiments

In the following, preferred aspects and examples will be described in more detail with reference to the accompanying figures. Same or similar features in different drawings and examples are referred to by similar reference numerals. It is to be understood that the detailed description below relating to various preferred aspects and preferred examples are not to be meant as limiting the scope of the present disclosure.

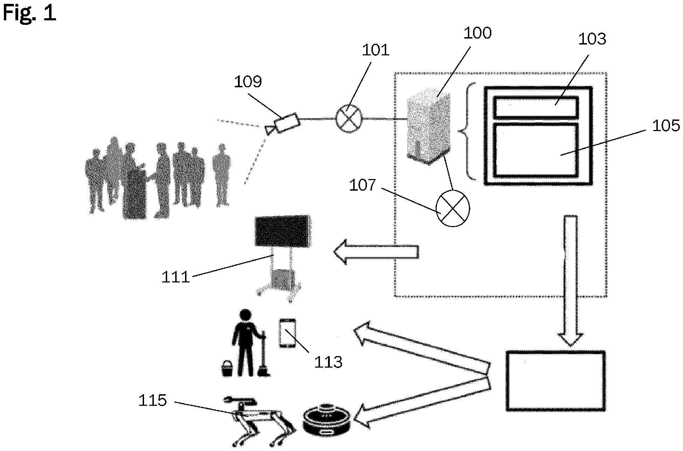

In Fig. 1 a monitoring system 100 is shown. The monitoring system 100 comprises an input interface 101, a processor 103, a memory unit 105 coupled to the processor 103, the memory unit 105 storing program instructions that are executed by the processor 103, and an output interface 107.

The input interface 101 is connected to a camera system 109 at a specific location to be monitored. The input interface 101 may be a network interface, such as a wireless network interface for communication with the camera system 109 over a communication network, such as the internet or a telephone network, for example.

The output interface may be connected to various output devices, such as a monitor 111, a mobile phone, 113, or a robot 115. The robot 115 may be a so-called loT-device, such as a vacuum cleaner controlled via the internet.

The camera system 109 is fixed at a specific location to be monitored, such as an airport, a station or a hospital and captures people regularly in a stream of images.

The monitoring system 100 receives the stream of images form the camera system 109 using the input interface 101. The memory unit 105 provides the processor 101 with instructions to recognize each person in each image of the stream of pictures and to track positions of the people's limbs.

Further, the memory unit 105 provides the processor 103 with instructions to detect whether a person touched an object or a location in a reference image provided to the monitoring system 100 by a supervisor, for example.

In case a person touched an object and/or a location shown in the reference image, the monitoring system generates at least one indicator that indicates the object or location touched by the person.

In order to indicate a likelihood of the touched object and/or location being infectious, i.e. contaminated with a virus or a bacterium, for example, the indicator may comprise a quantitative parameter, such as a color of a color scale or a number of a number scale, indicating a length of a time or a number of touches of an area with which the person or a plurality of persons touched the object and/or the location.

Using the output interface 107, the indicator is output. According to an example, the indicator is provided as a marker on the reference image, using the monitor 111 or the mobile phone 113, such that a sanitizing technician can identify the object and/or the location to be sanitized as a priority, or make a sanitize plan based on a contamination condition indicated by the quantitative parameter.

Alternatively, or additionally, the robot 113 is provided with control commands for cleaning the objects and/or locations indicated by the indicator using the output interface 107.

The monitor 111 may also be used to display the indicator to people at the specific location, such that passengers can see and recognize contaminated spots in the specific location, which will help them to avoid touching such contaminated spots.

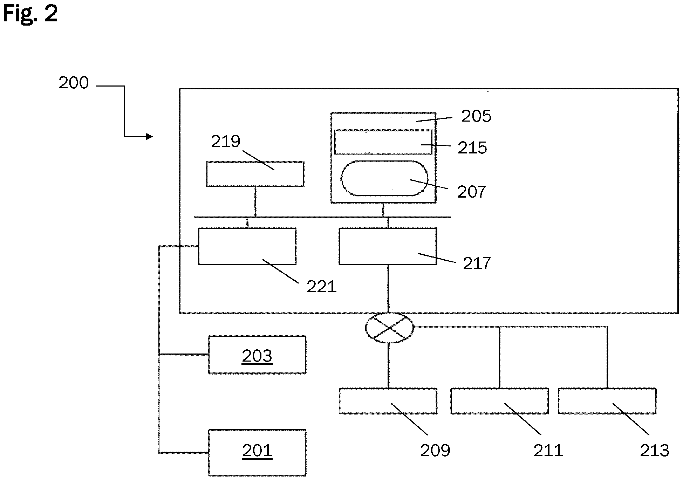

In Fig. 2 a configuration of a monitoring system 200 is shown. The monitoring system 200 comprises a video image photographing device 201 for capturing images, a video image storage device 203, a memory unit 205, and an output device, such as a display device 209, a mobile phone 211 or a robot 213.

The memory unit 205 stores a surface hygiene estimator 215 and a service hygiene tracking database 207 connected to an input interface 217 for receiving input images.

The surface hygiene estimator 215 stored in the memory unit 205 provides a processor 219 with commands for analyzing input images received via an input interface 221, identifying persons in the images, and detecting whether a person touches an object and/or a location in a reference image received via the input interface 221.

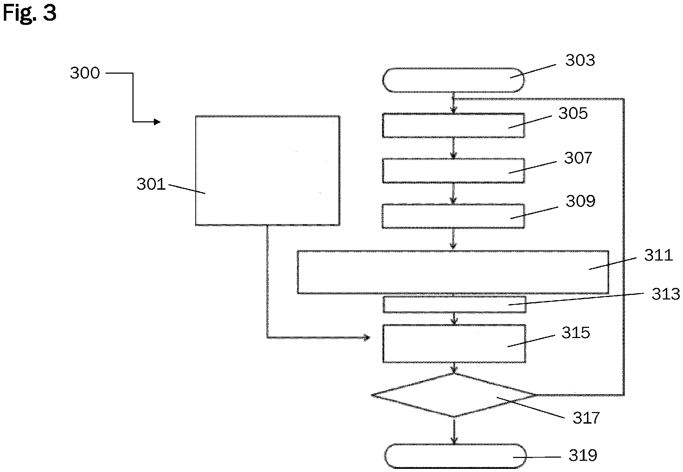

In Fig. 3 a flow chart 300 of a monitoring method according to an embodiment is shown. In a provisioning step 301, a reference image is provided. The reference image be annotated by a technician with highlighted areas and/or labels of objects to be monitored. Additionally, or alternatively an empty space in the reference image may be highlighted and/or annotated.

Alternatively, or additionally, the reference image may comprise depth information of an object or empty space in the specific location shown in the reference image. The depth information may be determined by a sensor, such as a LIDAR sensor, a RADAR sensor, or a stereo camera, for example.

In a starting step 303, a touch detect process is started, comprising an identification step 305 for identification of a person in a stream of pictures, an estimation step 307 for estimating a pose of the person and as assignment step 309 for assigning a person ID to the person and/or assigning a pose ID to the pose.

In an acquisition step 311 depth information and/or information about a movement of a limb of a person is acquired and stored in a database in a following storage step 313.

Based on the reference image provided in the provisioning step 301, a score is calculated in a calculation step 315, based on a difference between depth information of an object and/or location to be monitored in the reference image and depth information of a limb of the person stored in the storage step 313.

In a comparison step 317, the score calculated in the calculation step 315 is compared with a threshold. In case the score is greater that the threshold, no indicator is generated and the process starts over again with the identification step 305. In case the score is smaller or equals the threshold, an indicator is generated and the process ends with ending step 317.

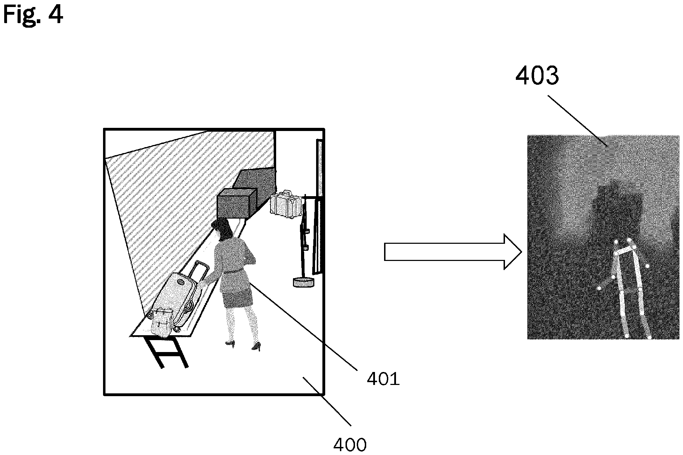

In Fig. 4, a pose identification process is visualized. In a first picture 400, a person 401 is shown. By using image processing, a body of the person 401 is reduced to lines in a processed image 403.

The processed image 403 may include colors according to a color scale of separating the lines from a background and for separating different lines representing different body parts from each other.

By following movement of the lines in the processed image, depth information of a limb of the person can be calculated and compared with depth information in a reference image.

In case a difference between the depth information of the lines and depth information of an object or a location in the reference image is smaller or equal to a given threshold, the object or the location is labelled as being touched by the person. Thus, the touched object or location is visualized by superimposing a touch marker on the reference image, or by ouputting the touched position itself, for example. If the locations and/or objects in the reference image are categorized, the monitoring system disclosed herein can also output a name of the locations and/or objects that have been touched.

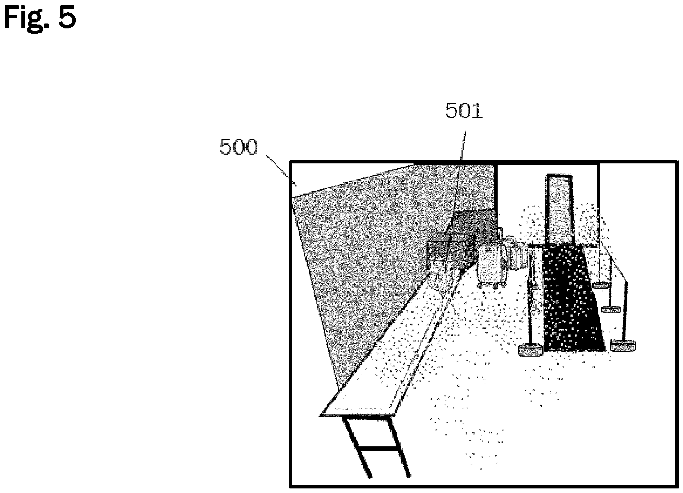

In Fig. 5 an example of a reference image 500 outputted with plurality of indicators 501 is shown. The indicators 501 indicate positions where a touching has been recognized by the monitoring system 100 as shown in Fig. 1, for example.

Each indicator of the indicators 501 may be color coded according to a color scale or enlarged by adding blur in order to represent a quantitative parameter of a corresponding touching. Thus, when the touching lasted very long, a large indicator and/or very red indicator may be shown.

Additionally, or alternatively, the color of particular indicators may be changed, if they correspond to different persons. Thus, a first group of indicators caused by touching of a first person can easily be separated from a second group of indicators caused by touching of a second person.

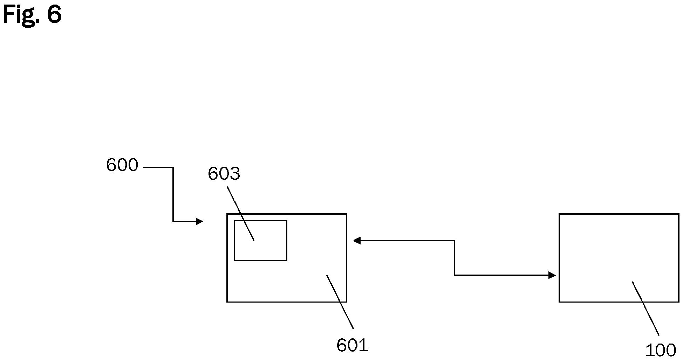

In Fig. 6, a cleaning system 600 is shown. The cleaning system 600 comprises a monitoring system 100, as shown in Fig. 1, for example, and a robot 601 with a sanitizing element 603.

The robot 601 is configured to sanitize an object indicated by an indicator, using the sanitzing element 603. Thus, the robot 601 receives an indicator or control commands based on an indicator from the monitoring system 100 and moves to a posiiton indicated by the indicator. Arrived at the position indicated by the indicator, the robot 601 activates the sanizing element 601 for sanitzing a location or an object.

As will be appreciated by one of skill in the art, the present disclosure, as described hereinabove and the accompanying figures, may be embodied as a method (e.g., a computer-implemented process or any other process), apparatus (including a device, machine, system, computer program product, and/or any other apparatus), or a combination of the foregoing.

Aspects/Examples of the present disclosure may be a software entirely (including firmware, resident software, micro-code, etc.), or a combination of software and hardware aspects that may be referred to as a "system". Furthermore, the present disclosure may take the form of a computer program product on a computer-readable medium having computer-executable program code embodied in the medium.

It should be noted that arrows may be used in drawings to represent communication, transfer, or other activity involving two or more entities. Double-ended arrows generally indicate that activity may occur in both directions (e.g., a command/request in one direction with a corresponding reply back in the other direction, or peer-to-peer communications initiated by either entity), although in some situations, activity may not necessarily occur in both directions.

Single-ended arrows generally indicate activity exclusively or predominantly in one direction, although it should be noted that, in certain situations, such directional activity actually may involve activities in both directions (e.g., a message from a sender to a receiver and an acknowledgement back from the receiver to the sender, or establishment of a connection prior to a transfer and termination of the connection following the transfer). Thus, the type of arrow used in a particular drawing to represent a particular activity is exemplary and should not be seen as limiting.

The present disclosure may be described with reference to flowchart illustrations and/or block diagrams of methods and apparatuses, and with reference to a number of sample views of a graphical user interface generated by the methods and/or apparatuses. It will be understood that each block of the flowchart illustrations and/or block diagrams, and/or combinations of blocks in the flowchart illustrations and/or block diagrams, as well as the graphical user interface, can be implemented by computer-executable program code.

The computer-executable program code may be provided to a processor of a general purpose computer, special purpose computer, or other programmable data processing apparatus to produce a particular machine, such that the program code, which executes via the processor of the computer or other programmable data processing apparatus, create means for implementing the functions/acts/outputs specified in the flowchart, block diagram block or blocks, figures, and/or written description.

The computer-executable program code may also be stored in a computer-readable memory unit that can direct a computer or other programmable data processing apparatus to function in a particular manner, such that the program code stored in the computer readable memory unit produce an article of manufacture including instruction means which implement the function/act/output specified in the flowchart, block diagram block(s), figures, and/or written description.

The computer-executable program code may also be loaded onto a computer or other programmable data processing apparatus to cause a series of operational steps to be performed on the computer or other programmable apparatus to produce a computer-implemented process such that the program code which executes on the computer or other programmable apparatus provides steps for implementing the functions/acts/outputs specified in the flowchart, block diagram block(s), figures, and/or written description. Alternatively, computer program implemented steps or acts may be combined with operator or human implemented steps or acts in order to carry out an embodiment of the disclosure.

It should be noted that terms such as "server" and "processor" may be used herein to describe devices that may be used in certain aspects of the present disclosure and should not be construed to limit the present disclosure to any particular device type unless the context otherwise requires. Thus, a device may include, without limitation, a bridge, router, bridge-router (brouter), switch, node, server, computer, appliance, or other type of device. Such devices typically include one or more network interfaces for communicating over a communication network and a processor (e.g., a microprocessor with memory unit and other peripherals and/or application-specific hardware) configured accordingly to perform device functions.

Communication networks generally may include public and/or private networks; may include local-area, wide-area, metropolitan-area, storage, and/or other types of networks; and may employ communication technologies including, but in no way limited to, analog technologies, digital technologies, optical technologies, wireless technologies (e.g., Bluetooth), networking technologies, and internetworking technologies.

It should also be noted that devices may use communication protocols and messages (e.g., messages created, transmitted, received, stored, and/or processed by the device), and such messages may be conveyed by a communication network or medium.

Unless the context otherwise requires, the present disclosure should not be construed as being limited to any particular communication message type, communication message format, or communication protocol. Thus, a communication message generally may include, without limitation, a frame, packet, datagram, user datagram, cell, or other type of communication message.

Unless the context requires otherwise, references to specific communication protocols are exemplary, and it should be understood that alternatives may, as appropriate, employ variations of such communication protocols (e.g., modifications or extensions of the protocol that may be made from time-to-time) or other protocols either known or developed in the future.

It should also be noted that logic flows may be described herein to demonstrate various aspects of the disclosure, and should not be construed to limit the present disclosure to any particular logic flow or logic implementation. The described logic may be partitioned into different logic blocks (e.g., programs, modules, functions, or subroutines) without changing the overall results or otherwise departing from the true scope of the disclosure.

Often, logic elements may be added, modified, omitted, performed in a different order, or implemented using different logic constructs (e.g., logic gates, looping primitives, conditional logic, and other logic constructs) without changing the overall results or otherwise departing from the scope of the disclosure.

The present disclosure may be embodied in many different forms, including, but in no way limited to, a graphical processing unit as well as computer program logic for use with a processor (e.g., a microprocessor, microcontroller, digital signal processor, or general purpose computer), programmable logic for use with a programmable logic device (e.g., a Field Programmable Gate Array (FPGA) or other PLD), discrete components, integrated circuitry (e.g., an Application Specific Integrated Circuit (ASIC)), or any other means including any combination thereof Computer program logic implementing some or all of the described functionality is typically implemented as a set of computer program instructions that is converted into a computer executable form, stored as such in a computer readable medium, and executed by a microprocessor under the control of an operating system. Hardware-based logic implementing some or all of the described functionality may be implemented using one or more appropriately configured FPGAs.

Computer program logic implementing all or part of the functionality previously described herein may be embodied in various forms, including, but in no way limited to, a source code form, a computer executable form, and various intermediate forms (e.g., forms generated by an assembler, compiler, linker, or locator).

Source code may include a series of computer program instructions implemented in any of various programming languages (e.g., an object code, an assembly language, or a high-level language such as Fortran, python, C, C++, JAVA, JavaScript or HTML) for use with various operating systems or operating environments. The source code may define and use various data structures and communication messages. The source code may be in a computer executable form (e.g., via an interpreter), or the source code maybe converted (e.g., via a translator, assembler, or compiler) into a computer executable form.

Computer-executable program code for carrying out operations of embodiments of the present disclosure may be written in an object oriented, scripted or unscripted programming language such as Java, Perl, Smalltalk, C++, or the like. However, the computer program code for carrying out operations of aspects of the present disclosure may also be written in conventional procedural programming languages, such as the "C" programming language or similar programming languages.

Computer program logic implementing all or part of the functionality previously described herein may be executed at different times on a single processor (e.g., concurrently) or may be executed at the same or different times on multiple processors and may run under a single operating system process/thread or under different operating system processes/threads.

Thus, the term "computer process" refers generally to the execution of a set of computer program instructions regardless of whether different computer processes are executed on the same or different processors and regardless of whether different computer processes run under the same operating system process/thread or different operating system processes/threads.

The computer program may be fixed in any form (e.g., source code form, computer executable form, or an intermediate form) either permanently or transitorily in a tangible storage medium, such as a semiconductor memory unit device (e.g., a RAM, ROM, PROM, EEPROM, or Flash-Programmable RAM), a magnetic memory unit device (e.g., a diskette or fixed disk), an optical memory unit device (e.g., a CD-ROM), a PC card (e.g., PCMCIA card), or other memory unit device.

The computer program may be fixed in any form in a signal that is transmittable to a computer using any of various communication technologies, including, but in no way limited to, analog technologies, digital technologies, optical technologies, wireless technologies (e.g., Bluetooth), networking technologies, and internetworking technologies.

The computer program may be distributed in any form as a removable storage medium with accompanying printed or electronic documentation (e.g., shrink wrapped software), preloaded with a computer system (e.g., on system ROM or fixed disk), or distributed from a server or electronic bulletin board over the communication system (e.g., the Internet or World Wide Web).

Hardware logic (including programmable logic for use with a programmable logic device) implementing all or part of the functionality previously described herein may be designed using traditional manual methods, or may be designed, captured, simulated, or documented electronically using various tools, such as Computer Aided Design (CAD), a hardware description language (e.g., VHDL or AHDL), or a PLD programming language (e.g., PALASM, ABEL, or CUPL).

Any suitable computer readable medium may be utilized. The computer readable medium may be, for example but not limited to, an electronic, magnetic, optical, electromagnetic, infrared, or semiconductor system, apparatus, device, or medium.

More specific examples of the computer readable medium include, but are not limited to, an electrical connection having one or more wires or other tangible storage medium such as a portable computer diskette, a hard disk, a random access memory unit (RAM), a read-only memory unit (ROM), an erasable programmable read-only memory unit (EPROM or Flash memory unit), a compact disc read-only memory unit (CD-ROM), or other optical or magnetic storage device.

Programmable logic may be fixed either permanently or transitorily in a tangible storage medium, such as a semiconductor memory unit device (e.g., a RAM, ROM, PROM, EEPROM, or Flash-Programmable RAM), a magnetic memory unit device (e.g., a diskette or fixed disk), an optical memory unit device (e.g., a CD-ROM), or other memory unit device.

The programmable logic may be fixed in a signal that is transmittable to a computer using any of various communication technologies, including, but in no way limited to, analog technologies, digital technologies, optical technologies, wireless technologies (e.g., Bluetooth), networking technologies, and internetworking technologies.

The programmable logic may be distributed as a removable storage medium with accompanying printed or electronic documentation (e.g., shrink wrapped software), preloaded with a computer system (e.g., on system ROM or fixed disk), or distributed from a server or electronic bulletin board over the communication system (e.g., the Internet or World Wide Web). Of course, some embodiments of the disclosure may be implemented as a combination of both software (e.g., a computer program product) and hardware. Still other aspects of the present disclosure are implemented as entirely hardware, or entirely software.

While certain exemplary aspects have been described and shown in the accompanying drawings, it is to be understood that such embodiments are merely illustrative of and are not restrictive on the broad disclosure, and that the aspects of the present disclosure are not limited to the specific constructions and arrangements shown and described, since various other changes, combinations, omissions, modifications and substitutions, in addition to those set forth in the above paragraphs, are possible.

Those skilled in the art will appreciate that various adaptations, modifications, and/or combination of the just described aspects and examples can be configured. Therefore, it is to be understood that, within the scope of the appended claims, the disclosure may be practiced other than as specifically described herein. For example, unless expressly stated otherwise, the steps of processes described herein may be performed in orders different from those described herein and one or more steps may be combined, split, or performed simultaneously. Those skilled in the art will also appreciate, in view of this disclosure, that different aspects or examples of the disclosure described herein may be combined to form other aspects or examples of the disclosure.

## Claims

1. A monitoring system (100, 200) for monitoring a surface, the monitoring system (100, 200) comprising:
- an input interface (101, 217, 221) configured to receive a reference image and a stream of pictures captured at a predetermined area,
- a processor (103, 219);
- an output interface (107);
- a memory unit (105, 205) coupled to the processor and storing program instructions that are executed by the processor,
wherein the program instructions comprise commands that configure the processor (103, 219) to:
- identify a person and its pose in the stream of pictures received by the input interface,
- extract a limb position of the person relative to the reference image received by the input interface, based on the pose,
- generate an indicator (501) that indicates a touched position based on the extracted limb position,
- output the indicator (501) via the output interface (107).

2. The monitoring system (100, 200) according to claim 1,
wherein the program instructions comprise commands that configure the processor (103, 219) to generate the indicator (501) in case a difference between a depth position of a limb of the person and depth information of the at least one object and/or location in the reference image is less than a given distance threshold.

3. The monitoring system (100, 200) according to claim 2,
wherein the program instructions comprise commands that configure the processor (103, 219) to calculate a score based on the difference between a depth position of a limb of the person and depth information of the at least one object and/or position in the reference image, and to choose an indicator (501) from a plurality of indicators according to the calculated score.

4. The monitoring system (100, 200) according to claim 2 or 3,
wherein the reference image includes depth information of at least one object and/or a locaation shown in the reference image or the program instructions comprise commands that configure the processor (103, 219) to calculate the depth information by comparing coordinates of objects to coordinates of empty space in the background of the reference image, or to calculate the depth information by using information provided by a depth sensor.

5. The monitoring system (100, 200) according to any of claims 2 to 4,
wherein the program instructions comprise commands that configure the processor (103, 219) to generate the indicator in case the difference between a depth position of a limb of the person and depth information of the at least one object and/or location in the reference image is less than a given distance threshold for a time-window that is larger than a given time threshold.

6. The monitoring system (100, 200) according to any of the preceding claims,
wherein the indicator (501) comprises at least one indicator information of the following list:
- a touch marker superimposing the reference image at the touched position,
- a position information of the touched position,
- a quantitative parameter of a sanitized state of the touched position,
wherein the quantitative parameter is a scale parameter determined by a given look-up table that associates a particular quantitative parameter to a particular contact time and/or a particular contact area of the person's limb and the touched position,
- a timestamp of a moment a touching of an object by a person was recognized.

7. The monitoring system (100, 200) according to any of the preceding claims, wherein the program instructions comprise commands that configure the processor (103, 219) to adapt the indicator over time according to a mathematical function representing a time during which a target substance becomes harmless for a human being.

8. The monitoring system (100, 200) according to any of the preceding claims,
wherein the indicator (501) is a coordinate of the reference image and/or a highlighted object in the reference image.

9. The monitoring system (100, 200) according to any of the preceding claims,
wherein the program instructions comprise commands that configure the processor to identify a particular person by storing a person ID in the memory unit.

10. The monitoring system (100, 200) according to any of the preceding claims,
wherein the program instructions comprise commands that configure the processor (103, 219) to track movement of a particular person using the person ID.

11. The monitoring system (100, 200) according to any of the preceding claims,
wherein the program instructions comprise commands that configure the processor (103, 219) to adapt a logic for generating the indicator (501) according to characteristics of the particular person using the person ID.

12. The monitoring system (100, 200) according to any of the preceding claims,
wherein the output interface (107) includes a display device (111).

13. A monitoring method (300) for monitoring a surface using the monitoring system (100, 200) according to any of claims 1 to 12, the monitoring method (300) comprising the follwing steps:
- receiving a reference image captured at a specific location using the input interface,
- receiving a stream of pictures captured at the specific location, using the input interface (101, 217, 221),
- identifying a person and its pose in the stream of pictures received by the input interface, using the processor (103, 219),
- extracting a limb position of the person relative to the reference image received by the input interface, based on the pose, using the processor (103, 219),
- generating an indicator (501) that indicates a touched position based on the extracted limb position, using the processor (103, 219),
- outputting the indicator (501) via the output interface (107), using the processor (103, 219).

14. A computer program product having computer program logic arranged to put into effect the monitoring method (300) for monitoring a surface according to claim 13, when being executed by a computer system.

15. A cleaning system (600) comprising a monitoring system (100, 200) according to any of claims 1 to 12 and a robot (601) with a sanitzing element (603),
wherein the robot (601) is confugured to sanitize an object indicated by an indicator, using the sanitzing element (603).
